# EUROPÄISCHE PATENTSCHRIFT

(11) **EP 2 509 499 B1**
(45) Veröffentlichungstag und Bekanntmachung des Hinweises auf die Patenterteilung: **18.09.2013**
(21) Anmeldenummer: 10778984.4
(22) Anmeldetag: 05.11.2010
(51) Int. Cl.: A61B 5/151

(54) **DETEKTIONSELEMENT ZUR POSITIONSBESTIMMUNG EINES LANZETTENELEMENTS IN EINER STECHHILFE**
DETECTION ELEMENT FOR DETERMINING THE POSITION OF A LANCET ELEMENT IN A LANCING DEVICE
ÉLÉMENT DE DÉTECTION POUR LA LOCALISATION D'UN ÉLÉMENT DE LANCETTE DANS UN ACCESSOIRE PERFORANT

(30) Priorität: 11.12.2009 DE 102009057892; 06.03.2010 DE 102010010507
(43) Veröffentlichungstag der Anmeldung: 17.10.2012
(73) Patentinhaber: Gerresheimer Regensburg GmbH, 93047 Regensburg (DE)
(72) Erfinder: STREHL, Michael, 92536 Pfreimd (DE)
(74) Vertreter: Hannke, Christian
(86) Internationale Anmeldenummer: PCT/EP2010/066911
(87) Internationale Veröffentlichungsnummer: WO 2011/069758

(56) Entgegenhaltungen:
- EP-A1- 1 614 383
- EP-A1- 1 913 872
- WO-A1-2007/104960
- WO-A2-2007/108967
- US-A1- 2007 293 883
- US-A1- 2009 099 431
- US-B1- 6 852 119

## Beschreibung

Die Erfindung betrifft ein Detektionselement für Lanzetten in Stechhilfen, gemäß dem Oberbegriff des Patentanspruches 1.

Bisher war es üblich, dass Lanzetten bzw. Lanzettenelemente, auch wenn sie innerhalb eines Lanzettenhalters innerhalb einer Stechhilfe zum Anstechen von Haut für eine Blutentnahme angeordnet sind, bei einem Lanzettenwechsel von Hand aus dem Gehäuse bzw. dem Lanzettenhalter der Stechhilfe herausgezogen werden mussten. Hierbei ist es problematisch, dass zum einen die Gefahr einer ungewollten Verletzung besteht und zum anderen blutbehaftete Lanzetten bzw. Lanzettenelemente von einem zuvor erfolgten Einstich angefasst werden müssen.

Zudem war es bei einem Auswechseln der Lanzette bzw. Lanzettenelement nicht möglich festzustellen, ob die Lanzette zuverlässig, d. h. bevorzugt zu 100 % in den Lanzettenhalter eingeführt worden ist bzw. an der vorgesehenen Stelle positioniert ist, so dass anschließend eine sichere Funktion der Stechhilfe, auch im Hinblick auf die Stechtiefe, nicht gewährleistet war.

Demzufolge ist die Aufgabe der Erfindung, ein Detektionselement für Lanzetten in Stechhilfen zur Verfügung zu stellen, welches signalisiert, dass ein Lanzettenelement nach erfolgtem Lanzettenwechsel vollständig wieder eingeführt ist.

Die Lösung der zuvor gestellten Aufgabe erfolgt erfingdungsgemäß mittels einer Blutlanzettenvorrichtung gemäss Anspruch 1.

Unter der Fixierposition wird zunächst jede Position des Lanzettenelements innerhalb bzw. im Bereich der Lanzettenaufnahmeeinrichtung verstanden, wobei bevorzugt die Fixierposition die optimale Position des Lanzettenelements in, an und/oder auf die Lanzettenaufnahmeeinrichtung zum Durchführen einer Penetrierbewegung darstellt. Besonders bevorzugt ist die Fixierposition eine Position in der das Lanzettenelement derart gegenüber der Lanzettenaufnahmeeinrichtung positioniert ist, dass ein definierter Stechvorgang durchführbar ist.

Die Blutlanzettenvorrichtung ist gemäß der vorliegenden Erfindung vorteilhaft, da sie einer Bedienperson ermöglicht zu erkennen, wann das ausgetauschte bzw. neu mit der Blutlanzettenvorrichtung gekoppelte Lanzettenelement in der optimalen Position bzw. der Fixierposition angeordnet ist.

In einer weiteren bevorzugten Ausführungsform der vorliegenden Erfindung ist die Detektionseinrichtung innerhalb dem Grundkörper angeordnet und gegenüber der Lanzettenaufnahmeeinrichtung bewegbar. Dies ist vorteilhaft, da zum einen durch die Anordnung der Detektionseinrichtung innerhalb des Grundkörpers die Detektionseinrichtung vor Beschädigung und Verschmutzung geschützt ist. Zudem unterstützt diese Anordnung die Einfachheit der Bedienung, da kein direktes Zusammenwirken des Benutzers bzw. der Bedienperson mit der Detektionseinrichtung erforderlich ist. Zum anderen ist dieses Merkmal vorteilhaft, da eine Bewegung der Detektionseinrichtung gegenüber der Lanzettenaufnahmeeinrichtung in einfacher Weise die Erzeugung von Signalen bevorzugt von Signalen bezüglich der Position des Lanzettenelements gegenüber der Lanzettenaufnahmeeinrichtung ermöglicht.

In einer weiteren bevorzugten Ausführungsform der vorliegenden Erfindung weist die Lanzettenaufnahmeeinrichtung mindestens eine Führung zum Führen der Detektionseinrichtung auf. Eine Führung wird hierbei bevorzugt als Gleitlager verstanden, das trocken oder geschmiert ausgeführt sein kann. Besonders bevorzugt ist eine Führung bzw. ein Gleitlager ein Kontaktbereich, insbesondere eine Aufliegefläche, eine Ausnehmung, eine Nut, ein Vorsprung, ähnliches und/oder Kombinationen daraus. Das Führen der Detektionseinrichtung erfolgt bevorzugt in der Verschieberichtung der Lanzettenaufnahmeeinrichtung, wobei ebenfalls denkbar ist, dass die Detektionseinrichtung auf einer dazu geneigten Bahn bzw. auf einer Kurvenbahn oder Kombinationen aus derartigen Bahnen geführt wird. Selbstverständlich ist ebenfalls denkbar, dass die Führung an dem Grundkörper, weiteren Einrichtungen und/oder durch das Zusammenwirken mehrere Einrichtungen und/oder dem Grundkörper gebildet wird.

Diese Ausführungsform ist vorteilhaft, da so eine definierte Ansteuerung der Detektionseinrichtung und somit eine genaue Signalausgabe bzw. Positionierung des Lanzettenelements gewährbar ist.

In einer weiteren bevorzugten Ausführungsform der vorliegenden Erfindung weist die Detektionseinrichtung ein Federelement zum zumindest zeitweisen Speichern von Energie auf, wobei die Energie im Wesentlichen schlagartig entladbar ist. Als Federelement werden in diesem Zusammenhang jegliche Bauteile verstanden, die geeignet sind Energie zu speichern und zu einem definierten Zeitpunkt bzw. in einer definierten Situation diese Energie wieder freigeben können. Die zumindest zeitweise Speicherung der Energie ist vorteilhaft, da vorstellbar ist, dass das Federelement die Energie zeitweise vollständig abgibt oder stets eine definierte Vorspannung aufweist. Schlagartig entladbar ist hierbei so zu verstehen, dass die Energie, die im Federelement gespeichert ist, sich innerhalb einer Zeit kleiner 2 Sekunden, bevorzugt < 1 Sekunden und besonders bevorzugt < 0,5 Sekunde nach dem Aufheben von Bauteilüberlagerungen, d. h. dem Freigeben des Federelements, entlädt bzw. teilweise entläd. Die Einleitung bzw. Entladung der Energie kann hierbei teilweise kontinuierlich oder teilweise diskontinuierlich erfolgen.

Diese Ausführungsform ist vorteilhaft, da selbst eine geringe Energie bei schlagartiger Entladung die Erzeugung eines von einer Bedienperson erfassbaren Signals ermöglicht.

In einer weiteren bevorzugten Ausführungsform der vorliegenden Erfindung ist die Energie mittels einer Kraftbeaufschlagung auf das Lanzettenelement bereitstellbar. Hierzu steht das Lanzettenelement mit der Detektionseinrichtung indirekt oder bevorzugt direkt in Kontakt, was eine zusätzliche Rückmeldung bewirkt, wenn die Fixierposition erreicht wurde, da das Einschieben des Lanzettenelements dann bevorzugt durch die Detektionseinrichtung unterbunden bzw. gebremst oder blockiert wird.

In einer weiteren bevorzugten Ausführungsform der vorliegenden Erfindung ist im Inneren des Grundkörpers, bevorzugt an der Lanzettenaufnahmeeinrichtung, eine Kraftaufnahmeeinrichtung, bevorzugt ein Keil, zum zumindest teilweisen Aufnehmen der in das Federelement eingeleiteten Kraft vorgesehen. Es ist hierbei ebenfalls denkbar, dass die Kraftaufnahmeeinrichtung innerhalb des Grundkörpers an der Wandung des Grundkörpers bzw. einer weiteren von der Lanzettenaufnahmeeinrichtung verschiedenen Einrichtung oder an der Lanzettenaufnahmeeinrichtung und einer beliebigen weiteren Einrichtung angeordnet ist. Eine Kraftaufnahmeeinrichtung dient bevorzugt als Widerstand, gegen den das Federelement zum Auslenken des Federelements bewegbar ist. Die Kraftaufnahmeeinrichtung kann abschnittsweise als Keil bzw. keilförmig und/oder kurvenförmige Abschnitte aufweisend ausgebildet sein.

Diese Ausführungsform ist vorteilhaft, da die Erzeugung bzw. Einleitung der zur Signalausgabe erforderlichen Energie beim Einsetzen des Lanzettenelements und somit durch einen ohnehin erforderlichen Arbeitsschritt möglich ist.

In einer weiteren bevorzugten Ausführungsform der vorliegenden Erfindung ist eine Fixier- und/oder Ausgabeeinrichtung zum Verändern der Kopplung zwischen der Lanzettenaufnahmeeinrichtung und dem Lanzettenelement sowie zum Auslenken des Federelements der Detektionseinrichtung vorgesehen. Die Fixier- und/oder Ausgabeeinrichtung dient bevorzugt zur manuellen Betätigung und weist dazu besonders bevorzugt ein Handbetätigungselement auf, wobei ebenfalls denkbar ist, dass die Fixier- und/oder Ausgabeeinrichtung oder einzelne Funktionen die durch die Fixier- und/oder Ausgabeeinrichtung ausgeführt werden durch weitere Einrichtungen manuell, automatisiert oder teilautomatisiert ausgeführt werden. Eine Veränderung der Kopplung zwischen der Lanzettenaufnahmeeinrichtung und dem Lanzet-tenelement bedeutet bevorzugt eine Veränderung eines Reib und/oder Formschlusses, der zwischen der Lanzettenaufnahmeeinrichtung und dem Lanzettenelement in gekoppeltem Zustand besteht. Es ist denkbar, dass mittels der Fixier- und/oder Ausgabeeinrichtung die Veränderung der Kopplung und die Auslenkung des Federelements zeitgleich, zeitversetzt oder zeitweise zeitgleich erfolgt.

Diese Ausführungsform ist vorteilhaft, da durch die Betätigung bzw. Bewegung, insbesondere durch das Verschieben der Fixier- und/oder Ausgabeeinrichtung mehrere Funktionen in einem Betätigungsgang, d. h. z. B. dem einmaligen Verschieben der Fixier- und/oder Ausgabeeinrichtung in einer Richtung, ausführbar sind.

In einer weiteren bevorzugten Ausführungsform der vorliegenden Erfindung weist die Fixier- und/oder Ausgabeeinrichtung zum Auslenken des Federelements ein bevorzugt keilförmiges erste Kontaktelement, das zum Auslenken des Schnappelement mit einem am Schnappelement ausgebildeten zweiten Kontaktelement in Kontakt bringbar ist, auf. Keilförmig beschreibt in diesem Zusammenhang bevorzugt einen gegenüber der Bauteilebene geneigten Anteil, der somit zumindest teilweise eben und/oder zumindest teilweise kurvenförmig ausgebildet sein kann. Es ist ebenfalls denkbar, dass das zweite Kontaktelement keilförmig ausgebildet ist. Bevorzugt ist wenigstens eins der Kontaktelemente keilförmig ausgebildet und die Bauteilebene ist bevorzugt aus der Längsrichtung und der Breitenrichtung oder Tiefenrichtung der Blutlanzettenvorrichtung gebildet.

Diese Ausführungsform ist vorteilhaft, da die Fixier- und/oder Ausgabeeinrichtung in einfacher Weise mit dem zweiten Kontaktelement und somit dem Schnappelement zum Auslenken des Schnappelements in Kontakt bringbar ist.

In einer weiteren bevorzugten Ausführungsform der vorliegenden Erfindung weist die Detektionseinrichtung einen sich merklich in einer Ebene erstreckenden Bauteilkörper auf, an dem das Federelement in Form eines zungenartigen Abschnitts gegenüber einem zungenartigen Versteifungsabschnitt ausgebildet ist, wobei sich im Bereich eines Endes des Federelements im Wesentlichen rechtwinklig zu der Bauteilebene das zweite Kontaktelement, das zumindest einen Kurvenabschnitt ausbildet, erstreckt. An dem Bauteilkörper ist bevorzugt eine Kontaktkante bzw. Kontaktfläche vorgesehen, mit der der Bauteilkörper mit dem Lanzettenelement in Kontakt bringbar ist.

Diese Ausführungsform ist vorteilhaft, da die Detektionseinrichtung ein Zwischenelement ausbildet, das in vorteilhafter Weise zumindest zeitweise bevorzugt mit einer Vielzahl an Bauteilen, wie z. B. der Lanzettenaufnahmeeinrichtung, dem Lanzettenelement und/oder der Fixier- und/oder Ausgabeeinrichtung indirekt oder bevorzugt direkt zusammenwirken kann.

Ein derartiges Detektionselement ist als ein Teil zu verstehen, welches als separates Bauteil ausgebildet ist und bevorzugt zeitweise direkt oder indirekt in Kontakt mit der Rückseite der Lanzette oder auch der Lanzettenhaltevorrichtung steht. Dieses separate Bauteil kann mittels eines Schnappverschlusses bzw. Schnappelements ein Geräusch beim Einsetzen des Lanzettenelements, insbesondere bei einem sich nach hinten bzw. nach innen gerichtetem Verschieben des Lanzettenelements erzeugen, wobei bevorzugt durch die Kraftaufbringung auf das Lanzettenelement das Detektionselement zumindest zeitweise verschiebbar ist. Somit erzeugt das Bauteil durch einen Schnappvorgang ein Geräusch, sobald das neu eingesetzte Lanzettenelement vollständig in die Blutlanzettenvorrichtung und damit in die Lanzettenaufnahmeeinrichtung bzw. den Lanzettenhalter eingeführt worden ist. Der Benutzer kann somit sicher sein, dass zu diesem Zeitpunkt die Lanzette vollständig mit der Lanzettenhaltevorrichtung verbunden ist.

Zudem wird hierdurch möglich, dass Lanzettenelemente verschiedenster Art, die beispielsweise keinen Stoppring im vorderen Bereich an ihrer Außenumfangsseite aufweisen, eingesetzt werden können, ohne dass die Gefahr besteht, dass die Lanzette nicht vollständig in der Lanzettenaufnahmeeinrichtung eingeführt ist.

Es ist ebenfalls vorstellbar, dass das Federelement nicht an der Detektionseinrichtung ausgebildet ist, sondern dass an der Detektionseinrichtung eine Kraftaufnahmeeinrichtung ausgebildet ist und das Federelement am Grundkörper der Blutlanzettenvorrichtung, der Lanzettenaufnahmeeinrichtung oder einem weiteren Bauteil innerhalb der Blutlanzettenvorrichtung angeordnet ist. Es ist ebenfalls denkbar, dass ein Schnappelement an dem Lanzettenelement ausgebildet ist.

Weitere Vorteile, Ziele und Eigenschaften vorliegender Erfindung werden anhand nachfolgender Beschreibung anliegender Zeichnungen erläutert, in welchen beispielhaft Blutlanzettenvorrichtungen sowie einzelne Bauteile von Blutlanzettenvorrichtungen dargestellt sind. Komponenten der Blutlanzettenvorrichtung, welche in den Figuren wenigstens im Wesentlichen hinsichtlich ihrer Funktion übereinstimmen, können der Übersichtlichkeit halber nicht in allen Figuren beziffert und erläutert sein.

Es zeigt:
- Fig. 1: eine aufgeschnittene Darstellung einer beispielhaften Stechhilfe gemäß einer Ausführungsform der vorliegenden Erfindung;
- Fig. 2: ein Kurvenelement das für den Einsatz in einer Blutlanzettenvorrichtung gemäß der vorliegenden Erfindung geeignet ist;
- Fig. 3: eine Lanzettenaufnahmeeinrichtung die für den Einsatz in einer Blutlanzettenvorrichtung gemäß der vorliegenden Erfindung geeignet ist;
- Fig. 4: ein Lanzettenelement, wie es beispielsweise in der vorliegenden Erfindung Einsatz finden kann;
- Fig. 5: eine beispielhafte perspektivische Teildarstellung einer Blutlanzettenvorrichtung gemäß der vorliegenden Erfindung in einer ersten Stellung;
- Fig. 6: eine beispielhafte erste Anordnung im zeitlichen Ablauf des Einsetzens einer neuen Lanzette;
- Fig. 7: eine beispielhafte zweite Anordnung im zeitlichen Ablauf des Einsetzens einer neuen Lanzette;
- Fig. 8: eine beispielhafte dritte Anordnung im zeitlichen Ablauf des Einsetzens einer neuen Lanzette;
- Fig. 9: eine beispielhafte Darstellung einer Ausschiebeanordnung; und
- Fig. 10: eine beispielhafte perspektivische Darstellung einer erfindungsgemäßen Detektionseinrichtung.

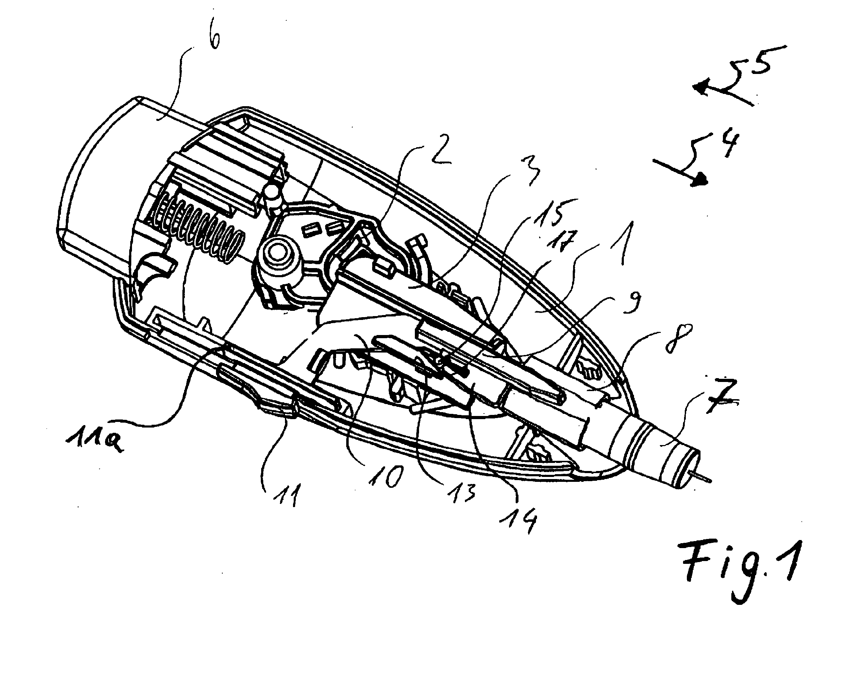

In Fig. 1 ist eine aufgeschnittene Darstellung einer beispielhaften Stechhilfe gemäß einer Ausführungsform der Erfindung gezeigt. Der obere Gehäuseanteil ist in dieser Darstellung nicht wiedergegeben.

Der untere Grundkörper 1 bzw. untere Gehäuseanteil weist u. a. ein Steuerkurvenelement 2 auf, das während des Stechvorgangs von einem hier nicht näher dargestellten Bolzen bevorzugt von der Lanzettenaufnahmeeinrichtung 3 abgelaufen wird, um die Lanzettenaufnahmeeinrichtung 3 in Vorwärtsrichtung 4 oder Rückwärtsrichtung 5 zu verschieben. Ausgelöst werden kann dieser Vorgang bevorzugt durch einen hier nicht dargestellten Druckknopf. Der rückwärtig angebrachte Druckknopf 6 dient zum Einbringen einer Kraft zum Vorspannen einer Feder, die direkt oder indirekt auf das Steuerkurvenelement 2 einwirkt.

Weiterhin ist der Fig. 1 ein Lanzettenelement 7 zu entnehmen, das in einem vorderen Abschnitt 8 der Lanzettenaufnahmeeinrichtung 3 angeordnet ist. In dem vorderen Abschnitt 8 der Lanzettenaufnahmeeinrichtung 3, der bevorzugt mindestens einen Spalt aufweist, ist in dieser Darstellung zumindest teilweise ein stabförmiges Element 9 mit einer keilförmigen Spitze zum Aufweiten des vorderen Abschnitts 8 eingeführt. Die Aufweitung bzw. Verformung des vorderen Abschnitt 8 bewirkt, dass ein Reibschluss und/oder Formschluss der zwischen dem vorderen Element 8 und dem Lanzettenelement 7 besteht verändert bzw. erzeugt oder aufgehoben wird.

Das stabförmige Element 9 ist bevorzugt integraler Bestandteil der Fixier- und/oder Ausgabeeinrichtung 10, wobei ebenfalls vorstellbar ist, dass das stabförmige Element 9 an der Fixier- und/oder Ausgabeeinrichtung 10 oder einer weiteren Einrichtung angebracht ist.

Die Fixier- und/oder Ausgabeeinrichtung 10 weist bevorzugt ein Handbetätigungselement 11 und eine Führungseinrichtung 11a auf. Das Handbetätigungselement 11 dient zum Verschieben der gesamten Fixier- und/oder Ausgabeeinrichtung 10 und die Führungseinrichtung 11a dient besonders bevorzugt zum Gewährleisten einer definierten Verschiebebewegung, wobei die Führungseinrichtung 11a mit einer weiteren Einrichtung und/oder dem Grundkörper in Form eines Gleitlagers bzw. Gleiten zusammenwirkt.

Ferner ist an der Fixier- und/oder Ausgabeeinrichtung 10 ein erstes Kontaktelement 13 ausgebildet, das eine bevorzugt keilförmige Spitze zum in Kontakt bringen mit einer Detektionseinrichtung 14, insbesondere mit einem zweiten Kontaktelement 15, das an der Detektionseinrichtung 14 ausgebildet ist, aufweist.

Das zweite Kontaktelement 15 ist in dieser Darstellung als Zapfenelement ausgebildet und erstreckt sich bevorzugt rechtwinklig zur Verschiebebewegung 4 und somit in Höhenrichtung des Grundkörpers 1. In Höhenrichtung ist unter dem zweiten Kontaktelement 15 an dem Federelement 17 bevorzugt mindestens ein zungenförmiges Laschen- bzw. Stabteil der Detektionseinrichtung 14, die besonders bevorzugt als Schnappeinrichtung ausgebildet, als Formschlusselement angebracht, das mit der Kraftaufnahmeeinrichtung 16 (in dieser Darstellung nicht gezeigt) zum Erzeugen einer Federkraft und/oder Fixierung, insbesondere einem Formschluss, bzw. zum Ausgeben oder Erzeugen eines Signals zusammenwirken kann.

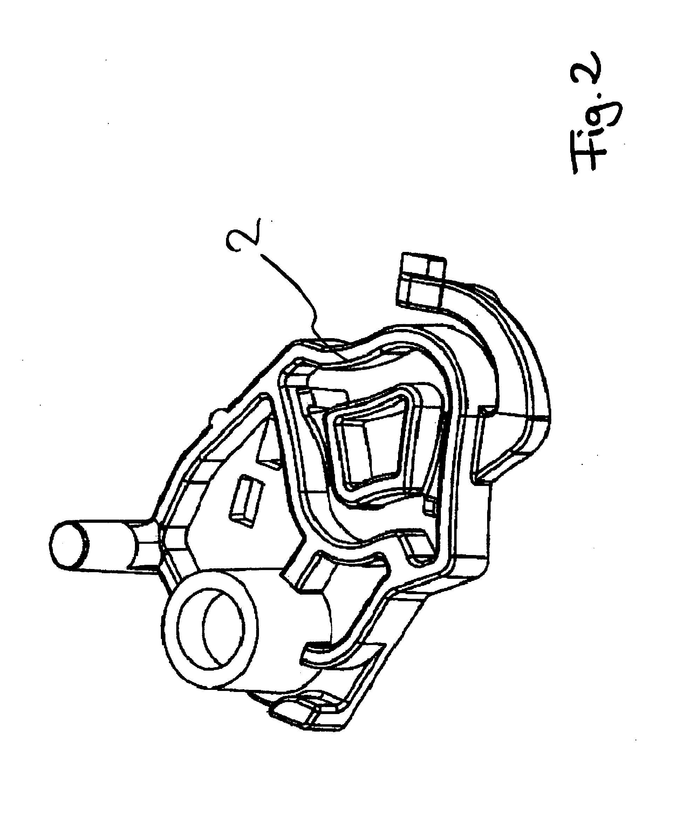

Der Fig. 2 ist eine beispielhafte Kurvenbahn zu entnehmen, die derart gestaltet ist, dass sie in Abhängigkeit einer beaufschlagten Kraft ein Verschieben der Lanzettenaufnahmeeinrichtung 3 und somit des Lanzettenelements 7 in Ausschieberichtung 4 und Einschieberichtung 5 bewirkt.

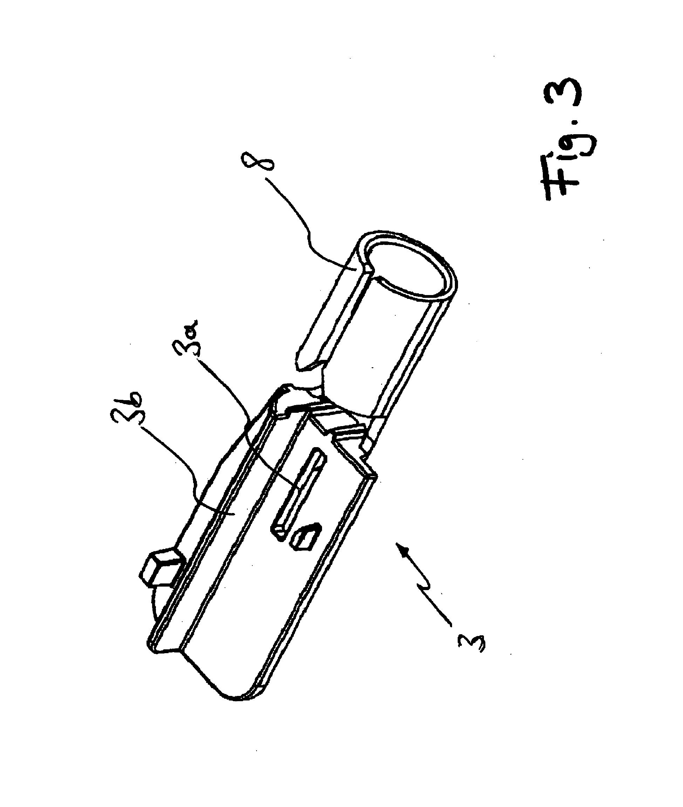

Der Fig. 3 ist eine beispielhafte Lanzettenaufnahmeeinrichtung 3 zu entnehmen. Die Lanzettenaufnahmeeinrichtung weist hierbei zumindest einen vorderen Abschnitt 8 zum Aufnehmen des Lanzettenelements 7 auf. Ferner umfasst die Lanzettenaufnahmeeinrichtung 3 eine erste Führung 3a, die bevorzugt zum Führen der Detektionseinrichtung 14 geeignet ist und eine zweite Führung 3b, die bevorzugt zum Führen bzw. in Kontakt bringen mit dem stabförmigen Element 9 geeignet ist.

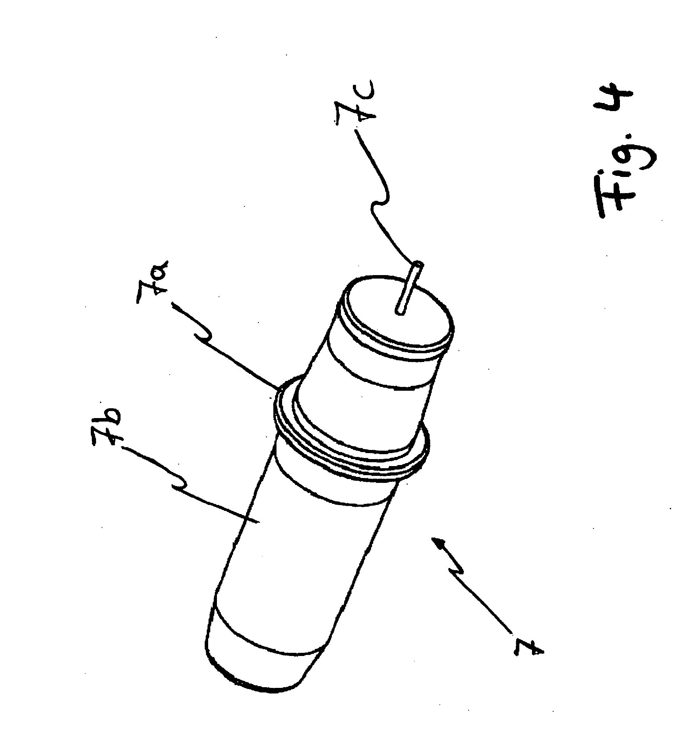

Der Fig. 4 lässt sich ein beispielhaftes Lanzettenelement 7 entnehmen, das einen Ring 7a an dem Lanzettenkörper 7b aufweist. Die Lanzette 7c ragt in Längsrichtung des Lanzettenkörpers 7b aus diesem heraus. Es ist hierbei vorstellbar, dass insbesondere Lanzettenelemente 7 mit und ohne den Ring 7a in der erfindungsgemäßen Blutlanzettenvorrichtung eingesetzt werden können.

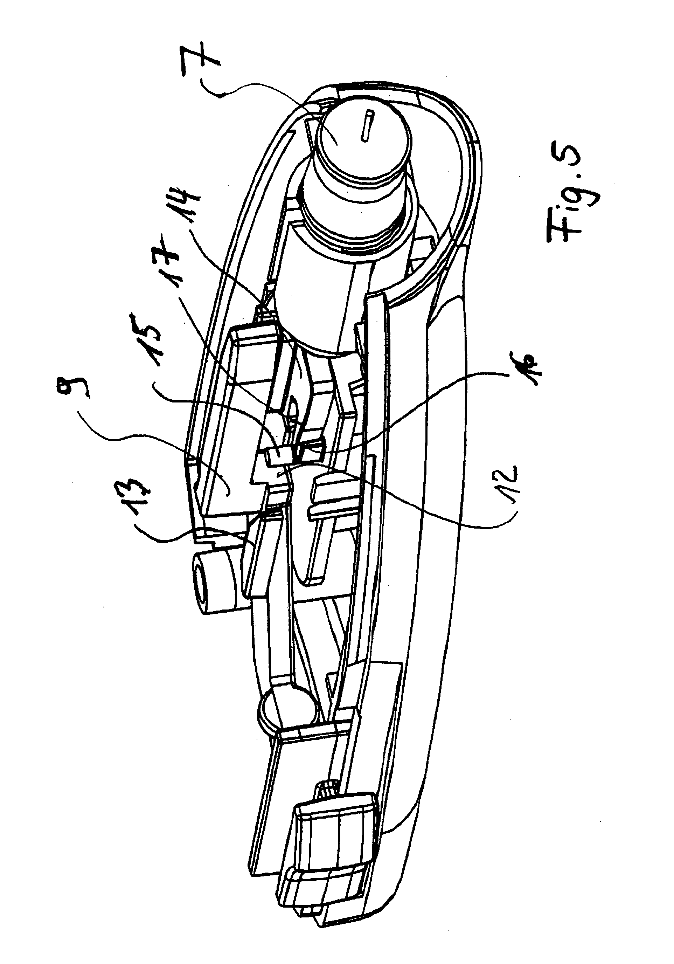

Der Fig. 5 ist ein keilförmig ausgebildetes erstes Kontaktelement 13 zu entnehmen, wobei dieses Kontaktelement 13 die Aufgabe besitzt, das das erfindungsrelevante Geräusch verursachende Bauteil, d. h. die Detektionseinrichtung 14, insbesondere das Federelement 17 mit einem daran angebrachten Vorsprung, der zapfenartig ausgebildet sein kann, aus einer Einrastbewegung bzw. Stellung herauszulösen.

Ein wesentlicher Punkt der Erfindung ist, dass das zweite Kontaktelement 15 bzw. der Zapfen 15 bei einem nach vorne Schieben der Fixier- und/oder Ausgabeeinrichtung 10 aus einer Verankerung gegenüber der Kraftaufnahmeeinrichtung 16, die beispielsweise als Vorsprung 16 ausgebildet ist, gelöst wird, was dazu führt, dass das Federelement 17, das beispielsweise als Zungenelement 17 der Detektionseinrichtung 14 ausgebildet ist, verformt bzw. ausgelenkt wird. Diese Auslenkung des Federelements 17 führt dazu, dass das zweite Kontaktelement 15 aus einer Arretierung gegenüber dem Vorsprung 16 bzw. der Kraftaufnahmeeinrichtung 16 herausgelöst wird und hierdurch die Detektionseinrichtung 14 zusammen mit der Fixier- und/oder Ausgabeeinrichtung 10 bevorzugt nach vorne bzw. in Richtung 4 verschoben wird.

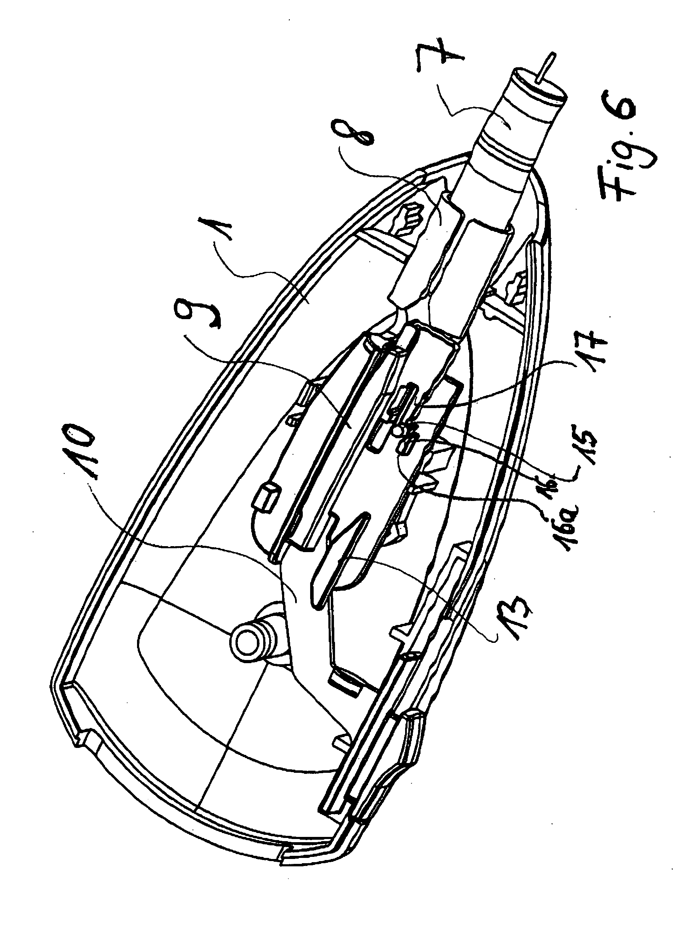

In den Figuren 6, 7 und 8 wird der zeitliche Ablauf des Einsetzen eines neuen Lanzettenelements 7 in die Lanzettenaufnahmeeinrichtung 3 und somit die Blutlanzettenvorrichtung bzw. Stechhilfe beispielhaft dargestellt.

Das Lanzettenelement 7 wird gemäß Fig. 6 in den zylinderförmigen vorderen Abschnitt 8 eingesetzt. In dieser Position ist das stabförmige Element 9 bevorzugt nach hinten bzw. in Richtung 5 verschoben. Ebenso ist die gesamte Fixier- und/oder Ausgabeeinrichtung 10 bevorzugt in der rückwärtigen Position angeordnet.

Das zweite Kontaktelement 15, das an dem Federelement 17 angebracht ist, ist noch nicht wieder in Eingriff gebracht, d. h. noch nicht an einer rückwärtigen Seite 16a der Kraftaufnahmeeinrichtung 16 angeordnet. Vielmehr befindet er sich in Vorwärtsrichtung 4 betrachtet vor der Kraftaufnahmeeinrichtung 16.

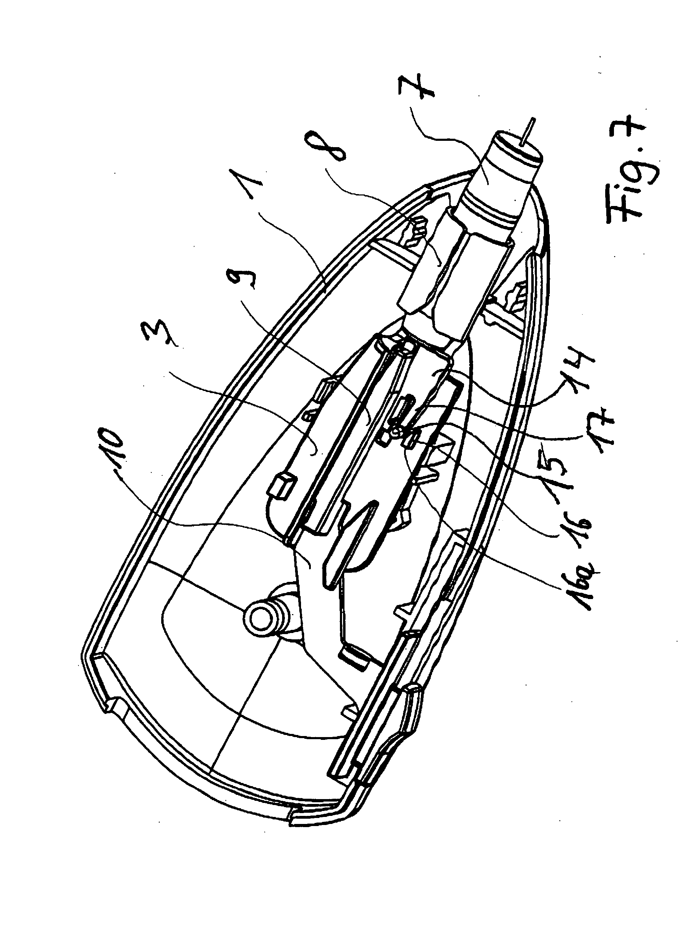

In Fig. 7 ist das zweite Kontaktelement 15 bereits ein Stück weiter in Richtung 5 verschoben. Zugleich ist die Detektionseinrichtung 14 mit der daran angebrachten Zunge bzw. dem daran ausgebildeten Federelement 17 nach innen bzw. in Richtung 5 verschoben. Dies erfolgt z. B. durch das Einführen des Lanzettenelementes 7 in eine noch weiter nach innen gerichtete Position innerhalb der Stechhilfe.

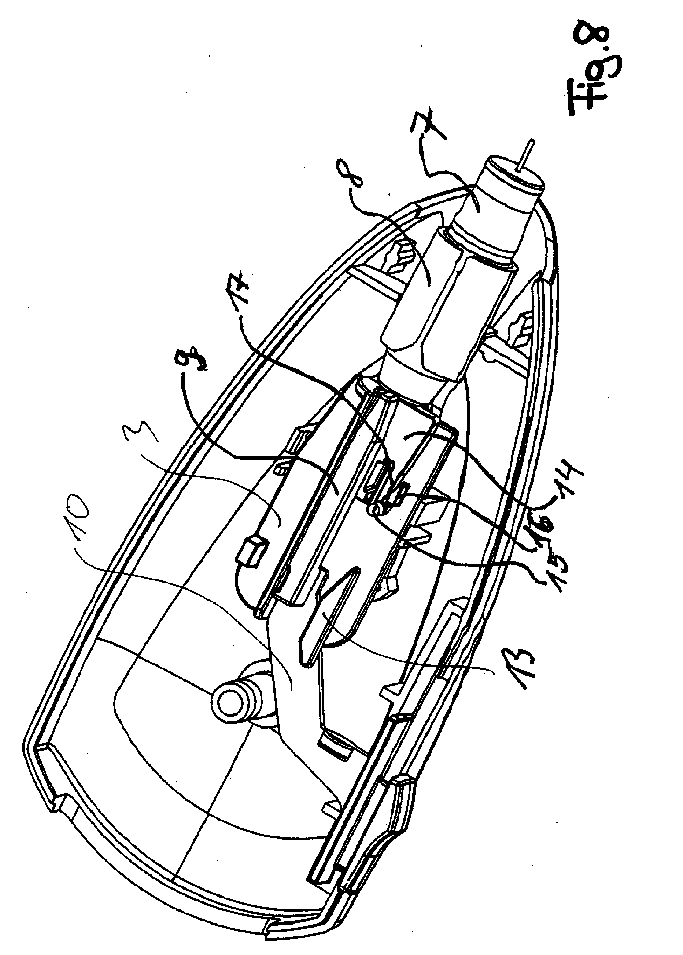

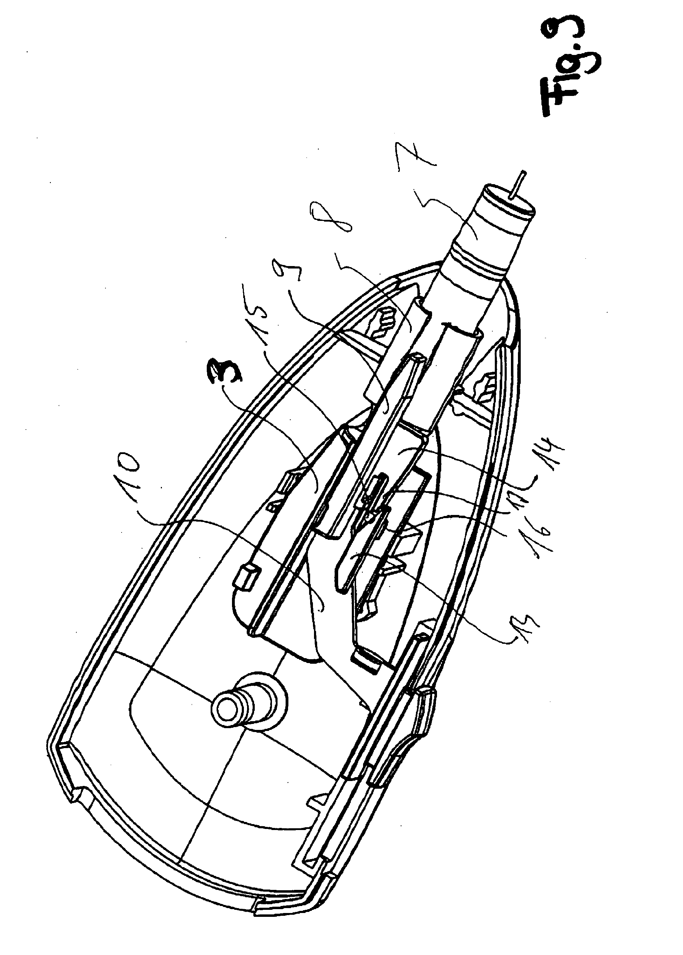

In Fig. 8 ist nun das Lanzettenelement 7 vollständig in die Lanzettenaufnahmeeinrichtung 3 eingesetzt. Die Detektionseinrichtung 14 ist hierbei vollständig nach hinten verschoben. Somit ist das zweite Kontaktelement 15 bzw. der darunter angeordnete bevorzugt einen Hinterschnitt ausbildende Abschnitt gegenüber der Kraftaufnahmeeinrichtung 16 in Eingriff gebracht, in dem er auf der Rückseite 16a der Kraftaufnahmeeinrichtung 16 positioniert ist. Diese Positionierung geschieht durch einen Entspannungsvorgang des Federelements 17, welches an der Detekitonseinrichtung 14 angebracht ist. Dieser Entspannungsvorgang bewirkt, dass das Federelement 17 in Richtung der Kraftaufnahmeeinrichtung 16 bewegt wird. Fig. 1 und Fig. 9 zeigen eine Position der Lanzette, der Lanzetteneinrichtung 3 und des Schiebeelements 10, bei der das Spreizelement 9 bereits das vordere zylinderförmige Teil 8 der Lanzettenhalteeinrichtung aufgespreizt hat und somit ein Entnehmen der Lanzette bzw. des Lanzettenelements 7 möglich ist.

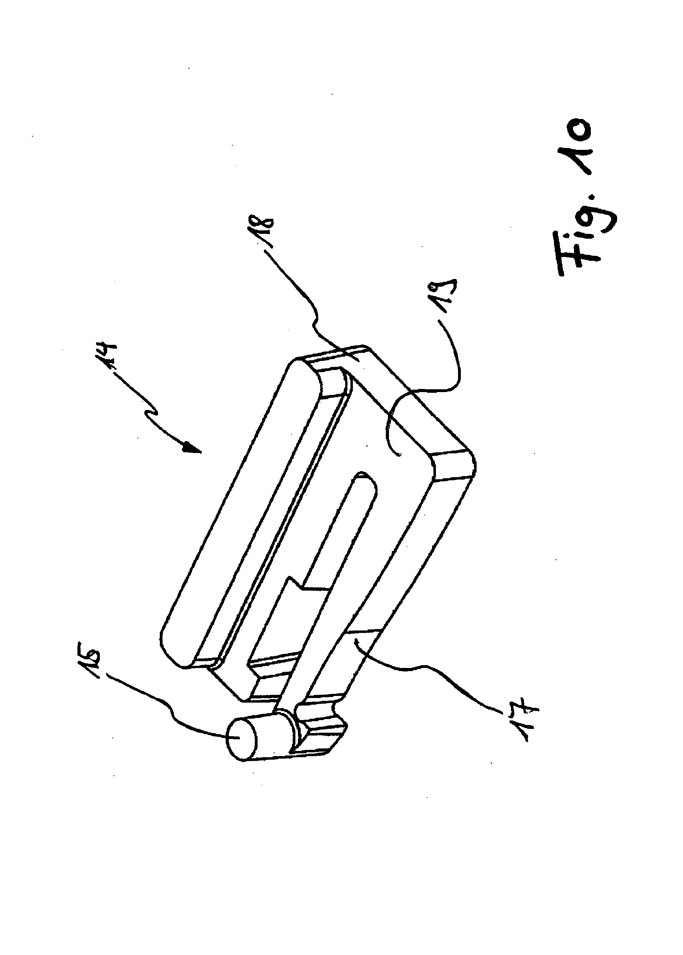

Das Federelement 17 weist unmittelbar vor dem zweiten Kontaktelement 15 eine Ausnehmung auf oder der Zapfen bzw. das zweite Kontaktelement 15 ist gegenüber dem restlichen Federelement 17 bezogen auf die Seitenfläche 19 kantig bzw. hervorstehend angeordnet. Dies geht aus Fig. 10 hervor, in der die Detektionseinrichtung 14 mit dem Federelement 17, dem die Detektionseinrichtung 14 bildenden Bauteilkörper 18 und dem zweiten Kontaktelement 15 separat dargestellt ist.

Durch eine derartige Anordnung der Detektionseinrichtung 14 kann vorteilhaft bei jedem Einstecken eines neuen Lanzettenelements bei einem zu erfolgenden Lanzettenwechsel zuverlässig eine Arretierung des Zapfens bzw. des zweiten Kontaktelements 15 gegenüber dem Vorsprung bzw. der Kraftaufnahmeeinrichtung 16 nur dann erhalten werden, wenn das Lanzettenelement 7 vollständig in die Lanzettenaufnahmeeinrichtung 3 eingeschoben worden ist. Dies wird vorteilhaft durch einen Sound bzw. ein Geräusch aufgrund des Einschnappvorgangs angezeigt. Der Benutzer weiß somit, dass das Lanzettenelement 7 vollständig in die Stechhilfe bzw. Blutlanzettenvorrichtung eingeführt worden ist.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind.

### Bezugszeichenliste

- 1: Grundkörper
- 2: Steuerkurvenelement
- 3: Lanzettenaufnahmeeinrichtung
- 3a: erste Führung
- 3b: zweite Führung
- 4: Ausschieberichtung / Vorwärtsrichtung
- 5: Einschieberichtung / Rückwärtsrichtung
- 6: Druckknopf
- 7: Lanzettenelement
- 7a: Ring
- 7b: Lanzettenkörper
- 7c: Lanzette
- 8: vorderer Abschnitt
- 9: stabförmiges Element
- 10: Fixier- und/oder Ausgabeeinrichtung
- 11: Handbetätigungselement
- 11a: Führungseinrichtung
- 12: Lanzettenschiebeteil
- 13: erstes Kontaktelement
- 14: Detektionseinrichtung
- 15: zweites Kontaktelement
- 16: Kraftaufnahmeeinrichtung
- 16a: Rückseite der Kraftaufnahmeeinrichtung
- 17: Federelement
- 18: Bauteilkörper
- 19: Seitenfläche

## Patentansprüche

1. Blutlanzettenvorrichtung mit einem Grundkörper (1) zum Anordnen einer Vielzahl an Vorrichtungskomponenten, umfassend eine Lanzettenaufnahmeeinrichtung (3) zum Koppeln der Blutlanzettenvorrichtung mit einem Lanzettenelement (7), mindestens ein Federelement (17) zum Federkraftbeaufschlagen der Lanzettenaufnahmeeinrichtung (3) und ein Betätigungselement zum Auslösen einer Bewegung der Lanzettenaufnahmeeinrichtung (3), wobei
im Bereich der Lanzettenaufnahmeeinrichtung (3) eine Detektionseinrichtung (14) zum zumindest zeitweisen Ausgeben zumindest eines Signals hinsichtlich einer Fixierposition des mit der Blutlanzettenvorrichtung zumindest zeitweise zumindest teilweise gekoppelten Lanzettenelements (7) vorgesehen ist,
**dadurch gekennzeichnet, dass**
die Detektionseinrichtung (14) zum Sichern ihrer Position gegenüber der Lanzettenaufnahmeeinrichtung (3) und/oder zum Ausgeben eines Signals ein Schnappelement als Bestandteil eines Federelements (17) zum Zusammenwirken mit einer Kraftaufnahmeeinrichtung (16) aufweist.

2. Vorrichtung nach Anspruch 1,
**dadurch gekennzeichnet, dass**
die Detektionseinrichtung (14) innerhalb dem Grundkörper (1) angeordnet ist und
gegenüber der Lanzettenaufnahmeeinrichtung (3) bewegbar ist.

3. Vorrichtung nach Anspruch 2,
**dadurch gekennzeichnet, dass**
die Lanzettenaufnahmeeinrichtung (3) mindestens eine Führung (3a) zum Führen der Detektionseinrichtung (14) aufweist.

4. Vorrichtung nach mindestens einem der vorangegangenen Ansprüche,
**dadurch gekennzeichnet, dass**
die Detektionseinrichtung (14) das Federelement (17) zum zumindest zeitweisen Speichern von Energie aufweist, wobei die Energie im Wesentlichen schlagartig entladbar ist.

5. Vorrichtung nach Anspruch 4,
**dadurch gekennzeichnet, dass**
die Energie mittels einer Kraftbeaufschlagung auf das Lanzettenelement (7) bereitstellbar ist.

6. Vorrichtung nach einem der vorangegangenen Ansprüche 4 oder 5,
**dadurch gekennzeichnet, dass**
im Inneren des Grundkörpers (1), bevorzugt an der Lanzettenaufnahmeeinrichtung (3), eine Kraftaufnahmeeinrichtung (16). bevorzugt ein Keil, zum zumindest zeitweisen zumindest teilweise Aufnehmen der in das Federelement (17) eingeleiteten Kraft vorgesehen ist.

7. Vorrichtung nach mindestens einem der vorangegangenen Ansprüche 4 bis 6,
**dadurch gekennzeichnet, dass**
eine Fixier- und/oder Ausgabeeinrichtung (10) zum Verändern der Kopplung zwischen der Lanzettenaufnahmeeinrichtung (3) und dem Lanzettenelement (7) sowie zum Auslenken des Federelements (17) vorgesehen ist.

8. Vorrichtung nach Anspruch 7,
**dadurch gekennzeichnet, dass**
die Fixier- und/oder Ausgabeeinrichtung (10) zum Auslenken des Federelements (17) ein keilförmiges erstes Kontaktelement (13) aufweist, das zum Auslenken des Schnappelements mit einem am Schnappelement ausgebildeten zweiten Kontaktelement (15) in Kontakt bringbar ist.

9. Vorrichtung nach mindestens einem der vorangegangenen Ansprüche,
**dadurch gekennzeichnet, dass**
die Detektionseinrichtung (14) einen sich merklich in einer Ebene erstreckenden Bauteilkörper (18) aufweist, an dem das Federelement (17) in Form eines zungenartigen Abschnitts gegenüber einem zungenartigen Verstelfungsabschnitt ausgebildet ist, wobei sich Im Bereich eines Endes des Federelements (17) rechtwinklig das zweite Kontaktelement (15) zumindest einen Kurvenabschnitt ausbildend, erstreckt.

## Claims

1. A blood lancet apparatus with a base body (1) for the arrangement of a plurality of apparatus components, comprising a lancet reception device (3) for coupling the blood lancet apparatus to a lancet element (7), at least one spring element (17) for acting upon the lancet reception device (3) with spring force and an actuating element for initiating a movement of the lancet reception device (3), wherein a detection device (14) for emitting at least for a time at least one signal with respect to a fixing position of the lancet element (7) coupled at least for a time and at least in part to the blood lancet apparatus is provided in the region of the lancet reception device (3), **characterized in that** for securing a position of the detection device (14) with respect to the lancet reception device (3) and/or for emitting a signal the detection device (14) has a snap element for cooperation with the force absorption device (16).

2. An apparatus according to claim 1, **characterized in that** the detection device (14) is arranged inside the base body (1) and is movable with respect to the lancet reception device (3).

3. An apparatus according to claim 2, **characterized in that** the lancet reception device (3) has at least one guide (3a) for guiding the detection device (14).

4. An apparatus according to at least one of the preceding claims, **characterized in that** the detection device (14) has the spring element (17) for storing energy at least for a time, wherein the energy is capable of being discharged substantially abruptly.

5. An apparatus according to claim 4, **characterized in that** the energy is capable of being supplied by means of an application of force upon the lancet element (7).

6. An apparatus according to at least one of the preceding claims 4 or 5, **characterized in that** a force absorption device (16), preferably a wedge, is provided in the interior of the base body (1), preferably on the lancet reception device (3), in order to absorb at least for a time and at least in part the force introduced into the spring element (17).

7. An apparatus according to at least one of the preceding claims 4 to 7, **characterized in that** a fixing and/or output device (10) is provided for changing the coupling between the lancet reception device (3) and the lancet element (7) and for deflecting the spring element (17).

8. An apparatus according to claim 7, **characterized in that** the fixing and/or output device (10) for deflecting the spring element (17) has a wedge shaped first contact element (13), which is capable of being brought into contact with a second contact element (15) formed on the snap element in order to deflect the snap element.

9. An apparatus according to at least one of the preceding claims, **characterized in that** the detection device (14) has a component body (18) which extends perceptibly in one plane and on which the spring element (17) is designed in the form of a tongue-like portion opposite a tongue-like reinforcement portion, wherein the second contact element (15), which forms at least one curved portion extends, at a right angle in the region of one end of the spring element (17).

## Revendications

1. Dispositif à lancette de prélèvement de sang avec un corps de base (1) destiné à agencer une pluralité de composants du dispositif, comportant un dispositif de réception de lancettes (3) pour coupler le dispositif à lancette de prélèvement de sang à un élément de lancettes (7), au moins un élément de ressort (17) destiné à solliciter la tension du ressort de l'unité de réception de lancettes (3), et un élément de manoeuvre pour déclencher un mouvement du dispositif de réception de lancettes (3),
un dispositif de détection (14) étant prévu dans la zone du dispositif de réception de lancettes (3) pour émettre au moins temporairement au moins un signal relatif à la position de fixation de l'élément de lancettes (7) couplée au moins temporairement, au moins partiellement, au dispositif à lancette de prélèvement de sang,
**caractérisé en ce que**
le dispositif de détection (14) présente, pour assurer sa position par rapport au dispositif de réception de lancettes (3) et/ou pour émettre un signal, un élément d'encliquetage faisant partie intégrante d'un élément de ressort (17) pour coopérer avec un dispositif d'absorption des forces (16).

2. Dispositif selon la revendication 1, **caractérisé en ce que** le dispositif de détection (14) est agencé à l'intérieur du corps de base (1) et mobile par rapport au dispositif de réception de lancettes (3).

3. Dispositif selon la revendication 2, **caractérisé en ce que** le dispositif de réception de lancettes (3) présente au moins un guidage (3a) destiné à guider le dispositif de détection (14).

4. Dispositif selon au moins l'une quelconque des revendications précédentes, **caractérisé en ce que** le dispositif de détection (14) présente l'élément de ressort (17) pour stocker l'énergie au moins temporairement, l'énergie pouvant être déchargée sensiblement soudainement.

5. Dispositif selon la revendication 4, **caractérisé en ce que** l'énergie peut être mise à disposition au moyen d'une sollicitation de force appliquée à l'élément de lancettes (7).

6. Dispositif selon l'une des revendications précédentes 4 ou 5, **caractérisé en ce qu'**à l'intérieur du corps de base (1), de préférence au niveau du dispositif de réception de lancettes (3), est prévu un dispositif d'absorption des forces (16), de préférence une cale, pour absorber au moins temporairement, au moins partiellement, la force introduite dans l'élément de ressort (17).

7. Dispositif selon au moins l'une quelconque des revendications précédentes 4 à 6, **caractérisé en ce qu'**un dispositif de fixation et/ou de distribution (10) est prévu pour modifier le couplage entre le dispositif de réception de lancettes (3) et l'élément de lancettes (7), ainsi que pour dévier l'élément de ressort (17).

8. Dispositif selon la revendication 7, **caractérisé en ce que** le dispositif de fixation et/ou de distribution (10), pour dévier l'élément de ressort (17), présente un premier élément de contact (13) en forme de cale qui, pour dévier l'élément encliquetable, est amené en contact avec un deuxième élément de contact (15), formé sur l'élément encliquetable.

9. Dispositif selon au moins l'une quelconque des revendications précédentes, **caractérisé en ce que** le dispositif de détection (14) présente un corps de composant (18), qui, de manière perceptible, s'étend dans un plan, sur lequel l'élément de ressort (17), sous la forme d'une partie en forme de languette, est formé en regard d'une partie de renfort en forme de languette, le deuxième élément de contact (15), formant au moins une partie courbe, s'étendant perpendiculairement dans la zone d'une extrémité de l'élément de ressort (17).
